# EUROPÄISCHE PATENTSCHRIFT

(11) **EP 1 282 448 B1**
(45) Veröffentlichungstag und Bekanntmachung des Hinweises auf die Patenterteilung: **30.03.2005**
(21) Anmeldenummer: 01940497.9
(22) Anmeldetag: 17.05.2001
(51) Int. Cl.: A61K 47/48, C07F 5/02, C07D 307/00, C07D 487/08

(54) **KONJUGAT AUF DER BASIS EINER HETEROBIFUNKTIONALEN BRÜCKENVERBINDUNG, DIE MIT EINEM IMMUNMODULATOR UND EINER ZELLERKENNENDEN EINHEIT SUBSTITUIERT IST**
HETEROBIFUNCTIONAL CROSS-LINKING AGENT CONJUGATE SUBSTITUTED BY AN IMMUNOMODULATOR AND CELL RECOGNITION UNIT
CONJUGUE A BASE D'UN AGENT DE RETICULATION HETEROBIFONCTIONNEL, SUBSTITUE PAR UN IMMUNOMODULATEUR ET UNE UNITE DE RECONNAISSANCE CELLULAIRE

(30) Priorität: 17.05.2000 DE 10024069
(43) Veröffentlichungstag der Anmeldung: 12.02.2003
(73) Patentinhaber: Glüsenkamp, Karl-Heinz, 45768 Marl (DE); Dilloo, Dagmar, 40629 Düsseldorf (DE)
(72) Erfinder: Glüsenkamp, Karl-Heinz, 45768 Marl (DE); Dilloo, Dagmar, 40629 Düsseldorf (DE)
(74) Vertreter: Graf von Stosch, Andreas, Dr. rer. nat.
(86) Internationale Anmeldenummer: PCT/EP2001/005672
(87) Internationale Veröffentlichungsnummer: WO 2001/087347

(56) Entgegenhaltungen:
- WO-A-96/06116
- DE-A- 4 205 306
- DE-A- 4 326 273
- DE-A- 19 600 707
- LETSINGER R L ET AL: "CHOLESTERYL-CONJUGATED OLIGONUCLEOTIDES: SYNTHESIS, PROPERTIES, ANDACTIVITY AS INHIBITORS OF REPLICATION OF HUMAN IMMUNODEFICIENCY VIRUS IN CELL CULTURE" PROCEEDINGS OF THE NATIONAL ACADEMY OF SCIENCES OF USA, NATIONAL ACADEMY OF SCIENCE. WASHINGTON, US, Bd. 86, Nr. 17, 1. September 1989 (1989-09-01), Seiten 6553-6556, XP000564447 ISSN: 0027-8424
- ZHANG J ET AL: "Linking Carbohydrates to Proteins Using N-(2,2-Dimethoxyethyl)-6-hyd roxy Hexanamide" TETRAHEDRON, ELSEVIER SCIENCE PUBLISHERS, AMSTERDAM, NL, Bd. 54, Nr. 39, 24. September 1998 (1998-09-24), Seiten 11783-11792, XP004133357 ISSN: 0040-4020

## Beschreibung

Die vorliegende Erfindung betrifft heterobifunktionale Brückenverbindungen (sogenannte "heterobifunctional cross-linkers"), die mit einem Immunmodulator und einer zellerkennenden Einheit substituiert sind, sowie ein Verfahren zu ihrer Herstellung sowie ihren Einsatz bei der ex-vivo-Zellmarkierung und derart markierte Zellen.

Verschiedene Strategien der Tumorbekämpfung sind nach dem Stand der Technik erprobt worden. So ist bspw. Interleukin-2, bekannt für seine co-stimulatorische Wirkung bei der Aktivierung von Immunzellen, insbesondere T-Zellen oder NK-Zellen, systemisch eingesetzt worden, um den Immunstatus des Tumorpatienten zu verbessern. Auf Grund der erheblichen systemischen Nebenwirkungen von Interleukin-2, auch gegenüber nicht entarteten Zellen, hat sich dieser Ansatz jedoch nicht als zielführend erwiesen.

Um eine spezifischer ansetzende Therapie auf der Basis von Cytokinen mit dem Ziel, deren immunmodulatorische Wirkung zu nutzen, zur Verfügung zu stellen, wurden im Stand der Technik Tumorzellen mit Cytokinen, bspw. auch IL-2, transfiziert. Derartig transfizierte Tumorzellen sollen in vivo durch erhöhte lokale IL-2-Expression in unmittelbarer Nachbarschaft des Tumorantigens eine tumorspezifische T-Zell-Antwort sicherstellen. Tatsächlich lassen sich im Mausmodell signifikante Verlängerungen der Überlebenszeit sowie eine Expansion und Aktivierung tumorspezifischer T-Zellen dadurch erreichen, daß Tumorzellen ex vivo durch eine Kombination von IL-2 und Lymphotaktin (zur Erhöhung der lokalen Konzentration von Immunzellen) mit Hilfe gentechnologischer Verfahren, d.h. insbesondere mit Hilfe viraler Vektoren, die die entsprechenden Sicherheitskriterien erfüllen, manipuliert wurden. Diese therapeutische Vorgehensweise wird in der Literatur auch als Tumorvakzination bezeichnet. Sie weist jedoch den Nachteil auf, daß viele primäre humane Tumorzellen mit den derzeit für die klinische Anwendung zugelassenen Vektoren nur mit geringer Effizienz transfiziert werden können. Hierzu kommt, daß die Langzeit-Nebenwirkungen auch solcher viraler Vektoren, die den entsprechenden Sicherheitsstandards genügen, nicht vorhersehbar sind. Auch bedarf es speziell ausgerüsteter Laboratorien, um derartige - im übrigen kostenintensive - virale Vektoren zur Verfügung zu stellen.

Um eine verzögerte Freisetzung von Cytokinen bei der Tumorbekämpfung sicherzustellen, wurden Cytokine in Kapseln eingebracht, deren Hülle eine Zusammensetzung aufweist, die erst sukzessiv unter gewissen physiologischen Bedingungen die Kapselinhaltsstoffe freisetzt. Derartige Kapseln wurden mit Tumorzellen gemischt und schließlich dieses Gemisch als Tumorvakzine subkutan injiziert. Diese Vorgehensweise vermag allerdings nicht zu gewährleisten, daß der für die Wirkung der Tumorvakzine verantwortliche Synergismus von Antigen präsentierender Tumorzelle und Cytokin enthaltender Kapsel, der auch die räumliche Nähe der beiden Komponenten voraussetzt, gegeben ist.

Wünschenswert wäre daher ein Vakzinierungssystem, das die räumliche Nähe der Komponenten sicherstellt.

Bekannt sind hierbei Ansätze, bei denen Cytokine an tumorspezifische Antikörper gekoppelt wurden. Derartige Komplexe oder auch cytotoxische Substanzen, gebunden an tumorspezifische Antikörper, wurden Tumorpatienten einerseits systemisch in der Hoffnung verabreicht, daß derartige Komplexe Tumorzellen, insbesondere auch metastasierte Zellen, erkennen können und sich derart Tumorzellen zielgerichtet bekämpfen lassen. Diese Therapie hat sich jedoch insofern als wenig geeignet erwiesen, als sich im Körper des Patienten nicht immer eine spezifische und intensive Anreicherung im Tumorbereich erzielen läßt und sich Patienten daher mit erheblichen, von diesen Komplexen ausgehenden Nebenwirkungen konfrontiert sahen. Auch ist bei direkter Kopplung des wirksamen Agens an den zellerkennenden Antikörper eine verzögerte Freisetzung von z.B. Cytokinen, die parakrin im Bereich der Tumorantigene präsentierenden Tumorzellen wirksam sind, nicht gewährleistet.

Um Wirkstoffe erst unter bestimmten physiologischen Bedingungen in aktiver Form freizusetzen, werden in der DE 43 26 273 A1 Verbindungen offenbart, die eine säurelabile Komponente aufweisen. Ein an die säurelabile Komponente angekoppelter Wirkstoff wird bevorzugt bei niedrigerem pH freigesetzt. Damit weist das Konjugat in der ungespaltenen Form den Charakter eines sogenannten "Prodrugs" auf.

Bei den in der DE 43 26 273 A1 offenbarten Konjugaten wird die Maskierung von biologisch aktiven Substanzen durch spezielle bicyclische Carbonsäureanhydride erreicht, wobei neuartige Wirkstoffe (Prodrugs) der nachfolgenden allgemeinen Formeln (Ia) bzw. (IIa) entstehen: 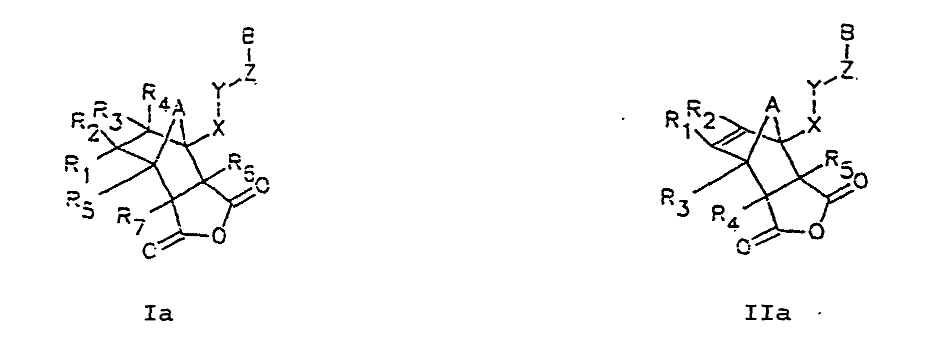 worin R¹ bis R⁴ bzw. R¹ und R² gleich oder verschieden sind und für Wasserstoff, gegebenenfalls substituiertes Cycloalkyl mit 3 bis 6 Kohlenstoffatomen oder geradkettiges oder verzweigtes Alkyl mit bis zu 8 Kohlenstoffatomen oder für gegebenenfalls substituiertes Phenyl stehen oder R¹ und R³ bzw. R² und R⁴ gemeinsam entweder einen 5- bis 7-gliedrigen ungesättigten Heterocyclus mit bis zu 3 Heteroatomen aus der Reihe N, S oder O oder einen 4- bis 6-gliedrigen Carbocyclus bilden, oder R¹ und R³ bzw. R² und R⁴ gemeinsam einen 3-gliedrigen Heterocyclus mit O oder NR¹⁰ bilden, worin R¹⁰ Wasserstoff oder geradkettiges oder verzweigtes Alkyl mit bis zu 6 Kohlenstoffatomen bedeutet,
X für ein Sauerstoff- oder Schwefelatom oder eine Gruppe der Formel -CR¹¹R¹²-, worin R¹¹ und R¹² gleich oder verschieden sind und für Wasserstoff, gegebenenfalls substituiertes Cycloalkyl mit 3 bis 6 Kohlenstoffatomen oder geradkettiges oder verzweigtes Alkyl mit bis zu 8 Kohlenstoffatomen stehen, oder für gegebenenfalls substituiertes Phenyl oder für Imidazolyl oder Indolyl stehen,
Y in Abhängigkeit von der jeweiligen Bedeutung von X für ein Sauerstoff- oder Schwefelatom oder für eine Gruppe der Formel -CO-, -CR¹³R¹⁴, -NR¹⁵- steht, worin R¹³, R¹⁴, R¹⁵ Wasserstoff oder geradkettiges oder verzweigtes Alkyl mit bis zu 6 Kohlenstoffatomen oder Phenyl bedeuten,
Z in Abhängigkeit von der jeweiligen Bedeutung von Y für eine direkte Bindung steht oder ein Sauerstoff- oder Schwefelatom sein kann oder für eine Gruppe der Formel -CO-, -CS-, -SO₂-, -CO-NR¹⁶, -CS-NR¹⁷-, P(O) (OR¹⁸)₂, -S- oder -CO-CH₂-CH₂-S-S- steht, worin R¹⁶, R¹⁷, R¹⁸ Wasserstoff oder geradkettiges oder verzweigtes Alkyl mit bis zu 6 Kohlenstoffatomen oder Phenyl bedeuten,
B in Abhängigkeit von der jeweiligen Bedeutung von Z für gegebenenfalls substituiertes Cycloalkyl mit 3 bis 6 Kohlenstoffatomen oder geradkettiges oder verzweigtes Alkyl mit bis zu 8 Kohlenstoffatomen oder für gegebenenfalls substituiertes Phenyl Imidazolyl, Indolyl, Thiomethyl, Thioethyl, 2-Pyridyl oder Adamantyl steht,
R⁵ die oben angegebene Bedeutung von R¹ bis R⁴ hat, mit diesen gleich oder verschieden ist, oder die gleiche Bedeutung wie X, Y, Z und B haben kann,
A für ein Sauerstoff- oder Schwefelatom oder für einen Rest der Formel NR¹⁹, worin R¹⁹ Wasserstoff oder geradkettiges oder verzweigtes Alkyl mit bis zu 8 Kohlenstoffatomen bedeutet, oder für einen Rest der Formel -CR²⁰R²¹-, worin R²⁰, R²¹ Wasserstoff oder geradkettiges oder verzweigtes Alkyl mit bis zu 6 Kohlenstoffatomen bedeuten und gleich oder verschieden sein können, oder für einen Rest der Formel -CR²²R²³ -CR²⁴R²⁵- steht, worin R²², R²³, R²⁴, R²⁵ gleich oder verschieden sein können, Wasserstoff oder Alkyl mit bis zu 6 Kohlenstoffatomen bedeuten,
R⁶ und R⁷ gleich oder verschieden sind und für Wasserstoff oder für geradkettiges oder verzweigtes Alkyl mit bis zu 8 Kohlenstoffatomen oder für Nitro, Halogen, Carboxy, Cyano stehen,
G für Hydroxy oder für eine über eine primäre oder sekundäre Aminfunktion gebundene Aminosäure und deren monomere oder polymere Derivate, wie z.B. Tryptophan, Tryptamin, N-Me-Tryptophan, N-Me-Tryptamin, Ditryptophan, Trypto-. phanmethylester oder für Aminogruppen enthaltende zytostatische Verbindungen, wie z.B. Melphalan, Nor-N-lost, cis-Platin bzw. Carboplatin oder andere Platinkomplexe, Anthracycline, wie z.B. Daunomycin, oder auch Mitomycin C und die verschiedenen Bleomycine; oder für literaturbekannte analgetische, antibiotische, anästhetische, antiphlogistische, antiseptische, antimykotische oder antivirale Wirkstoffe mit einer primären oder sekundären Aminogruppe steht;
und F die oben angegebene Bedeutung von G hat, wobei einer der Substituenten F oder G für Hydroxy stehen muß.

Diese in der DE 43 26 273 A1 offenbarten Verbindungen werden durch Addition des eine primäre oder sekundäre Aminogruppe enthaltenden Wirkstoffes an das bicyclische Säureanhydrid (I) bzw. (II) erhalten. Das bicyclische Säureanhydrid stellt demgemäß ein Maskierungsmittel dar. Die Bindung zwischen Wirkstoff und Maskierungsmittel ist eine labile Amidbindung, auf deren Stabilität durch gezielte chemische Modifikation in der Seitengruppe -X-Y-Z-B Einfluß genommen werden kann. Ferner übt der pH-Wert einen großen Einfluß auf die Stabilität der labilen Amidbindung aus. Die Verbindungen sind im pH-Bereich von 7,0 bis 7,5 von hoher Stabilität, jedoch im Bereich von pH 6,4 wesentlich labiler. Diese Verbindungen bzw. Konjugate stellen also nicht-toxische Vorläuferverbindungen (sogenannte "Prodrugs") von gängigen Arzneistoffen, insbesondere Zytostatika, dar.

Vorteilhaft sind derartige pH-labile "Prodrugs" insbesondere bei der Behandlung von Tumoren. Die pH-Abhängigkeit der labilen Amidbindung gestattet nämlich eine größere Tumorselektivität des maskierten Zytostatikums, da der pH in malignen Tumoren im Mittel bei 6,8 liegt, versus pH 7,1 im nicht-tumorigenen Gewebe. Diese Differenz wird durch Stimulation der aeroben Glycolyse maligner Zellen durch systemische Zufuhr von Glucose signifikant gesteigert (Mittelwert: pH 6,4), während sich die pH-Meßwertverteilung im normalen Gewebe nur wenig verändert. Damit bewirkt die pH-Abhängigkeit eine ortsspezifische und daher verzögerte Freisetzung des "Prodrugs" in aktiver Form. Das zur Maskierung der aktiven Komponente verwendete Maskierungsmittel wird daher auch als "slow-release-Komponente" bezeichnet.

Die in der DE 43 26 273 A1 offenbarten, säurelabilen "slow-release" Komponenten in Konjugaten maskieren in den Konjugaten in Form nicht-toxischer Vorläufer-Verbindungen bspw. herkömmliche Cytostatika, die erst nach Spaltung des labilen Amids ihre Zelltoxizität entfalten. Diese Konjugate sind jedoch für die Tumorvakzinierung nicht geeignet.

Weiterhin sind aus der DE 196 45 601 A1 Boronsäure-Derivate der Formel

Z-Y-B(OH)₂

bekannt, wobei Y ein substituiertes oder unsubstituiertes Alkylen des gesättigten unverzweigten oder verzweigten oder des ungesättigten Typs, vorzugsweise Propylen oder 2-Methyltrimethylen, oder ein substituierter cyclischer Rest des gesättigten heterocyclischen, alicyclischen oder aromatischen Typs, insbesondere Arylen, wie 1,3-Phenylen oder Benzylen, ist und
Z eine substituierte oder unsubstituierte biospezifische Verbindung ist.

Bei den in der DE 196 45 601 A1 offenbarten biospezifischen Verbindungen kann es sich um eine biologisch wirksame Verbindung, eine pharmazeutisch wirksame Verbindung, eine biochemisch wirksame Verbindung oder eine diagnostisch wirksame Verbindung handeln. Eine in dieser Druckschrift besonders bevorzugte biospezifische Verbindung ist ein Farbstoff, der in Abhängigkeit vom pH fluoresziert, vorzugsweise substituiertes Fluorescein. Bei diesen Boronsäure-Derivaten ist jedoch die Bindung zwischen biospezifischer Verbindung Z und dem Rest -Y-B(OH)₂ stabil.

Diese Boronsäuren können mit vicinalen OH-Gruppen auf eine Vielfalt von Substraten unter Bildung eines cyclischen Esters reagieren, welcher bei physiologischen pH-Werten ungewöhnlich stabil ist. Zellen enthalten eine Vielzahl von polymeren Kohlehydraten mit freien, vicinalen Diolgruppen und können demgemäß gezielt mit Boronsäuren markiert werden. Damit ist durch die in der DE 196 45 601 A1 beschriebenen Boronsäure-Derivate zwar die Markierung von Zellen mit Konjugaten offenbart, allerdings sind diese Markierungen für den zielgerichteten, therapeutischen Einsatz, insbesondere auch für die Tumorvakzinierung, bei Tumorpatienten ungeeignet.

Aufgabe der vorliegenden Erfindung ist es, Substanzen bzw. Verfahren zur Herstellung derartiger Substanzen zur Verfügung zu stellen, die es erlauben, auf kostengünstige Art eine effektive Tumorvakzinierung durchzuführen, wobei die Anforderungen an die Patientensicherheit bestmöglich gewährleistet sein sollen.

Die vorliegende Erfindung offenbart daher Konjugate, die einerseits mindestens einen labil gebundene Immunmodulator und andererseits eine Komponente aufweisen, die mittels spezifischer oder unspezifischer Wechselwirkung gezielt mit Zelloberflächen oder Zellteilen unter Bildung einer stabilen Bindung wechselwirken kann, wobei eine kontrollierte Freisetzungsrate des mindestens einen im Konjugat labil gebundenen Immunmodulators am Wirkort erreicht werden soll.

Die vorliegende Erfindung stellt damit zur Lösung der Aufgabe Verbindungen, enthaltend die Komponenten (1), (2) und (3) bereit, wobei Komponente (1) eine säurelabile heterobifunktionale Brückenverbindungen (sogenannter "cross-linker") ist, an die kovalent eine Komponente (2) mit Immunmodulatorfunktion und eine Komponente (3) mit Zelloberflächenbindungsfunktion angekoppelt sind, wobei die Bindung zwischen Komponente (1) und Komponente (2) säurelabil ist, insbesondere bei pH-Werten ≤ 6,8 labil ist.

In der vorliegenden Erfindung werden daher solche Verbindungen mit mindestens einer zellerkennenden und mindestens einer immunmodulatorischen Einheit bereitgestellt, die die allgemeinen Formeln (I) und (II) aufweisen, 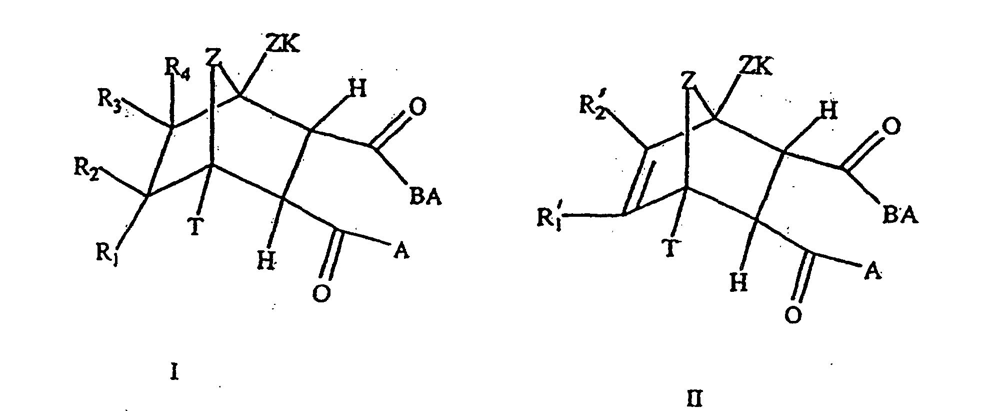 worin:
R₂ und R₄, für Wasserstoff oder gemeinsam für -O- oder-NR₁₀- stehen, worin R¹⁰ geradkettiges oder verzweigtes Alkyl mit bis zu 6 Kohlenstoffatomen ist,
R₁, R₃, R'₁ und R'₂ unabhängig voneinander Wasserstoff oder eine lineare, verzweigte oder cyclische, gegebenenfalls substituierte Alkylgruppe mit 3 bis 8 Kohlenstoffatomen stehen, wobei die Substituenten vorzugsweise aus Halogen, Amino, Cyano, Carboxy, geradkettigem oder verzweigtem Alkoxy mit bis zu 6 Kohlenstoffatomen oder Hydroxy ausgewählt sind;
oder R₁ und R₃ bzw. R'₁ und R'₂ gemeinsam entweder einen 5- bis 7-gliedrigen ungesättigten Heterocyclus mit bis zu 3 Heteroatomen aus der Reihe N, S oder O oder einen 4- bis 6-gliedrigen Carbocyclus bilden;
Z für Sauerstoff, Schwefel, -NR₅-, -CR₆R₇- oder -CR₆R₇-CR₈R₉- steht, worin R₅ für H oder C₁₋₈-Alkyl steht und R₆, R₇, R₈ und R₉ unabhängig für H oder C₁₋₆-Alkyl stehen;
A für Hydroxy steht;
BA für einen Rest einer immunmodulatorischen Substanz steht, die eine primäre oder sekundäre Aminfunktion enthält, wie beispielsweise für ein Cytokin;
ZK (Komponente 3) für eine mit dem Rest der allgemeinen Formeln I oder II kovalent verknüpfte zellerkennende Einheit mit spezifischer oder unspezifischer Zellerkennung steht, wobei als zellerkennende Einheiten alle niedermolekularen oder hochmolekularen, spezifischen oder unspezifischen zellerkennenden Einheiten in Frage kommen;
T unabhängig die Bedeutung von ZK aufweist oder für H oder eine lineare, verzweigte oder cyclische, gegebenenfalls substituierte Alkylgruppe mit 3 bis 8 Kohlenstoffatomen steht, wobei die Substituenten vorzugsweise Halogen, ein Amino-, Cyano-, Carboxy- oder Zuckerrest sind.

Damit löst der Gegenstand der vorliegenden Erfindung in vorteilhafter Weise die vorgenannte Aufgabe. Insbesondere erlaubt es die Struktur eines erfindungsgemäßen Konjugats, Immunmodulatoren so an die Zielzelle, insbesondere eine Tumorantigen präsentierende Zelle, ex vivo zu koppeln, daß eine - bevorzugt sukzessive - parakrine Freisetzung derselben in räumlicher Nachbarschaft zum Tumorantigen unter entsprechenden sauren Bedingungen des jeweiligen physiologischen Milieus in vivo erfolgen kann. Durch das Zusammentreffen der beiden immunologischen Aktivatorsignale (Tumorantigen und Immunstimulanz) kann die Aktivierung des Immunsystems, insbesondere also von Immunzellen, effektiv ablaufen.

Die zellerkennende Einheit (ZK) eines erfindungsgemäßen Konjugats kann spezifischer oder unspezifischer Natur sein. Ist sie spezifischer Natur, so wird sie vorzugsweise auf einer Rezeptor/Liganden-Wechselwirkung beruhen. Hierbei kann es sich bei der zellerkennenden Einheit in einem erfindungsgemäßen Konjugat sowohl um einen Rezeptor handeln, der einen membranständigen Liganden auf der Zielzelle erkennt, als auch um einen Liganden handeln, der mit einem membranständigen Rezeptor auf der Zielzelle in Wechselwirkung tritt. So etwa kann ein erfindungsgemäßes Konjugat als ZK einen, vorzugsweise löslichen, Fas-Liganden enthalten, der auf der Zielzelle an den Fas-Rezeptor andockt, oder es kann sich umgekehrt um den Fas-Rezeptor, vorzugsweise den Fas-Rezeptor ohne Transmembran- und intrazelluläre Abschnitte, handeln, der bei einem Kontakt des erfindungsgemäßen Konjugats mit einer Zielzelle die Bindung an einen membranständigen Fas-Liganden der Zielzelle vermittelt.

Auf diese Weise können die jeweiligen Bindungspartner aller physiologisch extrazellulär auf der Membran der Zielzelle auftretenden Rezeptoren oder Liganden als ZK in einem erfindungsgemäßen Konjugat zum Einsatz kommen. Ggf. können die Zielzellen durch Transfektion mit DNA, die für gewisse physiologisch auf den Zielzellen nicht oder in nur geringer Rate exprimierte, typischerweise membranständige Proteine codieren, auch so verändert werden, daß nunmehr auch die zu diesem transfizierten Protein gehörenden Bindungspartner als ZK in einem erfindungsgemäßen Konjugat in Betracht kommen. Die als ZK in einem erfindungsgemäßen Konjugat für die Ankopplung an Zielzellen vorzugsweise verwendeten biologischen Liganden oder Rezeptoren werden ganz besonders bevorzugt eine rekombinante Form, insbesondere eine auf die Bindungsdomäne(n) verkürzte Aminosäuresequenz, aufweisen.

Bevorzugt kann ein erfindungsgemäßes Konjugat multimerisiert, insbesondere dimerisiert, trimerisiert, tetramerisiert oder pentamerisiert, sein (bspw. durch kovalente und/oder nicht-kovalente Wechselwirkungen) und dann entsprechend auch als Dimer, Trimer, Tetramer oder Pentamer vorliegen. Die Multimerisierung kann über die Verknüpfung von Immunmodulatoren, heterobifunktionaler Brückenverbindung oder, bevorzugt, über die ZK erfolgen. Dabei kann ein erfindungsgemäßes Konjugat so aufgebaut sein, daß nur eine ZK (ZK1) einen Immunmodulator über einen säurelabilen "cross-linker" aufweist, während die weiteren Zellerkennungseinheiten mit ZK1 zwar di-, tri-, tetra- oder pentamerisieren, jedoch keine Immunmodulatoren bzw. "cross-linker" aufweisen. Alternativ können erfindungsgemäß aber auch Di-, Tri-, Tetra- oder Pentamere von erfindungsgemäßen Konjugaten, jeweils mit "cross-linker" und Immunmodulator gegeben sein, wobei die Di- bzw. Multimerisierung vorzugsweise über die jeweilige ZK, ggf. aber auch über die im Di- bzw. Multimer zusammengefaßten Immunmodulatoren oder die cross-linker, erfolgt. Durch die Di- oder Multimerisierung kann die Affinität und/oder Avidität von erfindungsgemäßen Konjugaten an die Zielzelle erhöht werden. Auch kann die Di- oder Multimerisierung dazu dienen, erfindungsgemäße Konjugate mit verschiedenen Immunmodulatoren in einem di- bzw. multimerisierten Komplex zusammenzufassen.

Ggf. können auch zwei erfindungsgemäße Konjugate mit mindestens einer ZK über einen Antikörper, der bspw. eine bestimmte Funktion auf dem "cross-linker" oder der ZK erkennt, zu einem Dimer eines erfindungsgemäßen Konjugats verbunden sein. Im Ergebnis führt diese Vorgehensweise dazu, daß über Antikörper vernetzte Konjugat-Dimere auf einer Zielzelloberfläche angekoppelt werden können.

Bei der Wahl der für die Zellerkennung verantwortlichen ZK ist für gewisse Anwendungen, insbesondere bei der herkömmlichen Tumorvakzinierung, zu berücksichtigen, daß der membranständige Bindungspartner nach Bindung der ZK im erfindungsgemäßen Konjugat nicht in die Zelle internalisiert wird. Es müssen daher für den Fall der Verwendung des Konjugats zur Tumorvakzinierung solche Rezeptor-Liganden-Systeme gewählt werden, die gewährleisten, daß eine Inkorporation des Komplexes unterbleibt und damit eine Freisetzung des Immunmodulators in den extrazellulären Raum erfolgen kann.

Alternativ kann es aber auch bspw. bei der Tumorvakzinierung erwünscht sein, daß der Immunmodulator in die Zelle eingeschleust wird. In diesem Fall wird erfindungsgemäß die ZK so gewählt, daß nach Bindung eines erfindungsgemäßen Konjugats an den Bindungspartner auf der Zielzelloberfläche eine Inkorporation des Konjugat-Rezeptor-Komplexes erfolgt und der Immunmodulator im intrazellulären Raum freigesetzt wird. Typischerweise wird es sich in diesem Fall bei dem Immunmodulator um ein Protein handeln, das als Genaktivator für bspw. Cytokine oder andere immunologisch relevante Proteine wirkt, bspw. durch direkte oder indirekte Bindung an regulatorische Einheiten auf der DNA oder sogenannte "Enhancer"-Bereiche.

Als ZK kommen vorzugsweise aber auch Antikörper oder Antikörperfragmente (bspw. Fab-Fragmente) bzw. deren Derivate in Betracht. Die Antikörper oder Antikörperfragmente erkennen typischerweise Strukturen auf der Zielzelloberfläche, bspw. sind als Epitope extrazelluläre Proteinabschnitte oder extrazelluläre Kohlenhydrate (membranständig oder an ein Protein gekoppelt), insbesondere für die jeweiligen Zielzellen typische Tumor-Antigene, zu nennen.

Lektine als ZK können in diesem Zusammenhang für die Bindung an die Zielzelloberfläche ebenso in Betracht kommen wie auch allgemein Kohlenhydratverbindungen. Auch einer der beiden Bindungspartner der (Strept)Avidin-Biotin-Bindungsreaktion ist typischerweise als ZK in einem erfindungsgemäßen Konjugat geeignet. Wie bereits zuvor erwähnt, sind Bindungspartner eines zellmembranständigen Rezeptors oder Liganden bzw. ein Fragment oder Derivat einer solchen Substanz ganz besonders bevorzugt.

Als unspezifische ZK in einem erfindungsgemäßen Konjugat können Liposomen, hydrophobe Anker (mit der Eigenschaft, sich in die Zellmembran einzulagern), wie z.B. Kohlenwasserstoffketten, oder lipidähnliche Moleküle, wie z.B. Choline, eingesetzt werden, und, besonders bevorzugt, werden vorliegend (Aryl)boronsäure-Derivate oder (Aryl)boronsäureester-Derivate offenbart. Hierbei wird die gesamte diesbezügliche Offenbarung aus der Druckschrift DE 196 45 601 A1 in die vorliegende Anmeldung einbezogen. Insbesondere können die in den Ansprüchen 1 und 2 der vorgenannten Druckschrift beschriebenen (Aryl)boronsäure-Derivate und die in den Ansprüchen 7, 8 und/oder 9 in derselben Druckschrift beschriebenen (Aryl)boronsäureester-Derivate als ZK in einem erfindungsgemäßen Konjugat zum Einsatz kommen. Insbesondere kann ZK für ZE- (B)ₙ- stehen, worin ZE ein Rest einer zellerkennenden Einheit (s. DE 196 45 601) ist, n=1 ist und B für eine Borgruppe steht und die zellerkennende Einheit ZE kovalent entweder an die Brückengruppe und damit mittelbar an die typischerweise als heterobifunktionaler "cross-linker" fungierende bicyclische Anhydridgruppe oder direkt an diese bicyclische Anhydridgruppe geknüpft ist.

Als besonders vorteilhaft für die Zellerkennung eines erfindungsgemäßen Konjugats erweist sich die Einführung von zwei zellerkennenden Systemen in der allgemeinen Formel (I) oder (II), wie beispielsweise zwei Arylboronsäuren.

Insbesondere in Hinblick auf die Ankopplung eines erfindungsgemäßen Konjugats an Zielzellen wird hierdurch eine besonders lang anhaltende Anhaftung eines erfindungsgemäßen Konjugats, das über eine heterobifunktionale Brückenverbindung säurelabil einen Immunmodulator maskiert, auf der Zielzellenoberfläche sichergestellt.

Als säurelabile heterobifunktionale Brückenverbindungen im erfindungsgemäßen Konjugat kommen solche Substanzen in Betracht, die einerseits eine stabile Bindung an eine ZK aufweisen, andererseits aber eine säurelabile Bindung an eine weitere Verbindung, bspw. einen Immunmodulator, ausbilden können. D.h. insbesondere, daß die Bindung des bspw. Immunmodulators an die Brückenverbindung bei einem pH-Wert von bspw. ≥ 7,4 stabil ist, während bei einem niedrigeren pH-Wert, insbesondere bei einem pH-Wert von ≤ 6,5, eine an die Brückenverbindung bzw. den "cross-linker" angekoppelte Verbindung freigesetzt wird. Hierbei wird es sich typischerweise um bicyclische Amide handeln, an die zum Erhalt eines erfindungsgemäßen Konjugats der Immunmodulator über eine Aminfunktion gebunden ist. Insbesondere bezieht die vorliegende Erfindung all jene cross-linker in ihre Offenbarung ein, die auch in der DE 42 05 306 A1 offenbart sind. Hierbei sind vor allem jene Brückenverbindungen zu nennen, die die Merkmale der in den Ansprüchen 1, 2 und 3 der DE 42 05 306 A1 beschriebenen Verbindungen aufweisen.

Ganz besonders bevorzugt sind jedoch als Bestandteile der erfindungsgemäßen Konjugate bicyclische Anhydride, wie in der DE 43 26 273 A1 offenbart. Die Offenbarung der DE 43 26 273 A1 wird daher in Hinblick auf die dort offenbarten bicyclischen Anhydride als Maskierungsmittel vollinhaltlich in die vorliegende Anmeldung einbezogen. Vor allem jene cross-linker, die durch die Merkmale der Ansprüche 1, 2 und 3 beschrieben werden, sind als cross-linker für die Immunmodulatoren einerseits und die ZK andererseits in einem erfindungsgemäßen Konjugat einsetzbar.

Um den Immunmodulator an einen heterobifunktionalen, bicyclischen "cross-linker", der vorzugsweise durch seine Anhydrid-Funktion den Immunmodulator maskiert, koppeln zu können, sollte der Immunmodulator vorzugsweise eine primäre oder sekundäre Aminfunktion enthalten. Die auf diese Weise sich mit dem "cross-linker" ergebende Amidbindung ist labil und wird vorzugsweise bei pH-Werten von ≤ 6,8, besonders bevorzugt ≤ 6,5 und ganz besonders bevorzugt ≤ 6,4 gespalten.

Unter den Immunmodulatoren eines erfindungsgemäßen Konjugats werden die Cytokine bevorzugt, insbesondere die Cytokine IL-1, IL-4, IL-6, IL-7, IL-2, INF-γ, TNF-α oder GM-CSF. Insbesondere IL-2 ist bevorzugt, da IL-2 sowohl cytotoxische T-Zellen als auch NK-Zellen aktivieren kann. Aber auch Chemokine, wie bspw. GROa ("Growth related Oncogene α"), IL-8 (Interleukin 8), MIP-2 ("Macrophage Inflammatory Protein 2"), IP-10 (Interferon-γ induzierbares Protein 10), RANTES ("Regulated on Activation, Normal T cell expressed and secreted"), MIP1α ("Macrophage Inflammatory Protein 1α"), MCP-1 ("Monocyte Chemoattractant Protein 1"), EOTAXIN oder Ltn (Lymphotactin), fallen im Sinne der vorliegenden Erfindung unter die Gruppe der Immunmodulatoren. Auch alle unter die Termini "Monokine", "Lymphokine" oder "Kolonie stimulierende Faktoren" fallenden Signalmoleküle sind als Immunmodulatoren in einem erfindungsgemäßen Konjugat bevorzugt. Auch die unter der Bezeichnung "Interferone" zusammengefaßten Faktoren stellen Immunmodulatoren dar. Weiterhin gehören zu den Immunmodulatoren auch co-stimulierende Oberflächenmoleküle von Zellen des Immunsystems oder Zellen mit Regulatorfunktion für das Immunsystem. Bspw. kann es sich bei dem Immunmodulator eines erfindungsgemäßen Konjugats um die Oberfächenmoleküle CD40L, B7.1, CD80, CD86, CD70 und/oder ICAM oder weitere Zelladhäsionsmoleküle. Schließlich werden im Rahmen der vorliegenden Erfindung auch Proteine als Immunmodulatoren bezeichnet, die mittelbar oder unmittelbar in die Regulation von am Immungeschehen beteiligten Proteinen (bspw. die vorgenannten Substanzen) eingreifen. Bei diesen Regulatorproteinen kann es sich bspw. um Aktivatoren oder Inhibitoren auf der Ebene der DNA-Transkription oder der Translation handeln.

Die Immunmodulatoren können stimulatorisch, vorzugsweise spezifisch auf gewisse Agenzien, z.B. spezisch zur Bekämpfung von Tumorzellen oder zur Bekämpfung von Bakterien bzw. virusinfizierten Zellen, wirken oder aber auch inhibitorisch, bspw. zur Attenuierung oder Inhibition überschießender Immunreaktionen, bspw. im Fall von Autoimmunerkrankungen.

Alle vorgenannten Immunmodulatoren können in einem erfindungsgemäßen Konjugat typischerweise in ihrer physiologischen Form vorliegen. Allerdings können auch Derivate oder Fragmente der physiologischen Aminosäuresequenzen bevorzugt sein, wobei zu berücksichtigen ist, daß die physiologische Wirksamkeit der eingesetzten Derivate oder Fragmente aufrechterhalten bleibt. Bei den Fragmenten wird es sich typischerweise um C- oder N-terminal verkürzte Sequenzen handeln, aber auch nicht-terminale Deletionen sind denkbar. Bevorzugt sind dabei Deletionen zwischen 1 und 50 Aminosäuren, ganz besonders bevorzugt zwischen 1 und 10 Aminosäuren. Unter Derivaten von Immunmodulatoren werden insbesondere Sequenzen mit einer oder mehr konservativen Aminosäuresubstitutionen verstanden, also bspw. der Austausch einer polaren gegen eine andere polare Aminosäure, einer hydrophoben oder aromatischen gegen eine andere hydrophobe bzw. aromatische Aminosäure.

Aber auch gegenüber den physiologischen Sequenzen, insbesondere N- oder C-terminal, verlängerte Immunmodulatoren werden erfindungsgemäß offenbart. Die Sequenzverlängerungen können funktionalen Charakter haben. Bspw. können Hybride aus zwei oder mehr Immunmodulatoren der vorgenannten Art, ggf. getrennt duch Linkerbereiche, fusioniert sein und als Immunmodulator-Hybrid in einem erfindungsgemäßen Konjugat auftreten. Auf diese Weise können in einem erfindungsgemäßen Konjugat mehrere biologische bzw. immunologische Funktionen so zusammengefaßt werden, daß eine optimale Wirkung eines derartigen Konjugats auf einer Zielzelle, bspw. in Hinblick auf seine Eigenschaften als Bestandteil einer Tumorvakzine, erreicht wird.

Darüber hinaus können Derivate von Immunmodulatoren oder Immunmodulator-Hybriden durch die synthetische Ankopplung von organischen Molekülen hergestellt werden. Hierbei kann es sich um Markierungs- oder Diagnosemittel, bspw. auch Kontrastmittel, handeln, die es erlauben die Tumorvakzine durch bildgebende Verfahren in vivo zu lokalisieren oder, bspw. durch Fluoreszenzmarker, bei Zellversuchen in vitro durch Einsatz entsprechender technischer Hilfsmittel, bspw. von Fluoreszenzmikroskopen, sichtbar zu machen. Ein oder mehr organische/s Molekül/e kann/können nicht nur an den Immunmodulator, sondern auch an den "cross-linker" und/oder an die ZK im erfindungsgemäßen Konjugat angekoppelt sein. Auch kann ein erfindungsgemäßes Konjugat mit mehreren, ggf. verschiedenen, organischen Molekülen derivatisiert werden.

Ggf. können auch eine oder mehr cytotoxische Substanzen, insbesondere auch Chemotherapeutika, zur Derivatisierung eines erfindungsgemäßem Immunmodulators durch kovalente Bindung an eine funktionelle Gruppe eines erfindungsgemäßen Immunmodulators eingesetzt werden. Insbesondere werden erfindungsgemäß auch solche Konjugate offenbart, die zwei oder mehr säurelabile "cross-linker", gebunden an nur eine ZK, aufweisen, wobei ein "cross-linker" einen Immunmodulator, ein Derivat oder Fragment desselben mit der ZK verbindet, während ein oder mehr andere, vorzugsweise in ihrer Säurestabilität unterschiedliche "cross-linker" ein oder mehr weitere Verbindungen mit derselben ZK konjugieren. So etwa können verschiedene Immunmodulatoren über jeweils einen "cross-linker" (gleicher oder unterschiedlicher Art) auf eine ZK gebracht werden.

Es können aber auch auf diese Weise Chemotherapeutika mit Immunmodulatoren in einem erfindungsgemäßen Konjugatmolekül kombiniert werden, bspw. das Chemotherapeutikum über eine säurestabilere und der Immunmodulator über eine säurelabilere Bindung. Ein derartiges erfindungsgemäßes Konjugat ist insbesondere dann vorteilhaft, wenn das Konjugat auf eine Tumorzelle als Tumorvakzine aufgebracht wird. Hierbei wird zunächst der Immunmodulator, gebunden an einen labileren cross-linker, freigesetzt, so daß die Vakzinierungsreaktion unter Beteiligung der Tumorzelle, des Immunmodulators und der zu aktivierenden Effektorzelle ablaufen kann. Verzögert wird dann, idealerweise nach Ablauf der Immunreaktion, das Chemotherapeutikum freigegeben, um eine zusätzliche, durch die räumliche Nachbarschaft mit hoher Wahrscheinlichkeit lokalspezifisch gegen Tumorzellen gerichtete cytotoxische Reaktion bei der systemischen Applikation zu ermöglichen. Statt des Chemotherapeutikums können aber verzögert auch Liganden freigesetzt werden, die lokal, vorzugsweise im Tumorgewebe, eine apoptotische Reaktion hervorrufen. Zu denken wäre bspw. an den (löslichen) Fas-Liganden.

Ein weiterer Gegenstand der vorliegenden Erfindung sind Verfahren zur Herstellung von Konjugaten der erfindungsgemäßen Art. Die Herstellung der erfindungsgemäßen Konjugate geht von einem konjugierten, mit einer oder zwei zellerkennenden Einheiten substituierten 5- oder 6-gliedrigen Ring-Dien, d.h einem Furan, Pyrrol, Cyclopentadien oder konjugiertem Cyclohexadien aus. An dieses Dien wird in einer Diels-Alder-Reaktion Maleinsäureanhydrid addiert. Die verbleibende Doppelbindung im Diels-Alder-Produkt (ungesättigten Maleinsäureanhydrid-Addukt) kann gegebenenfalls hydriert oder mit einer Peroxysäure oder einem Nitren umgesetzt werden, um das gesättigte, epoxiderte bzw. iminierte Maleinsäureanhydrid-Addukt zu ergeben. Das gesättigte oder ungesättigte Maleinsäureanhydrid-Addukt wird dann mit einer bioaktiven Substanz, insbesondere mit einem Immunmodulator, inkubiert, die ein primäres oder sekundäres Amin enthält, was die gewünschte Verbindung der Formel I oder II ergibt.

Mit den erfindungsgemäßen Verbindungen bzw. Konjugaten ist es zum ersten Mal gelungen, immunmodulatorisch wirkende Substanzen durch eine labile Bindung an eine bicyclische Verknüpfungsgruppe mit einer zellerkennenden Einheit zu knüpfen, wodurch eine kontrollierte Freisetzung der Wirksubstanzen am Wirkort erreicht wird. Diese neue Vorgehensweise eröffnet ein unerwartetes und breites biochemisches und medizinisches Anwendungsspektrum, insbesondere auch die Verwendung derartiger erfindungsgemäßer Konjugate für die Tumorvakzination und für die systemische Therapie.

Konjugate der erfindungsgemäßen Art oder Zusammensetzungen, enthaltend derartige Konjugate, können auch als solche, ggf. systemisch auf oralem oder parenteralem Weg, appliziert werden. Bevorzugt ist die Applikation der Konjugate oder der Zusammensetzungen, enthaltend derartige Konjugate, unmittelbar in die neoplastische Läsion, sofern dieselbe zugänglich ist. Ganz besonders bevorzugt ist daher die Injektion erfindungsgemäßer Zusammensetzungen oder Konjugate direkt in den Tumor, bspw. in Hauttumore, vor allem zur Behandlung des Melanoms. Die systemische Verabreichung wird auch bevorzugt sein, wenn die erfindungsgemäßen Konjugate oder Zusammensetzungen, enthaltend derartige Konjugate, zur Bekämpfung von Tumorerkrankungen mit Metastasen, Mikrometastasen oder vereinzelten dislozierten Tumorzellen eingesetzt werden.

Aber auch die Verwendung zur Behandlung bzw. zur Herstellung eines Azneimittels zur Behandlung von Autoimmunerkrankungen, insbesondere diesbezüglich zur gezielten Inhibition der Immunfunktion, und zur Behandlung von Infektionserkrankungen, insbesondere zur gezielten Stimulation der Immunabwehr, ist eine weitere Indikation für den vorliegenden Erfindungsgegenstand.

Ein weiterer Erfindungsgegenstand ist ein Addukt aus einer Zielzelle, an die ein erfindungsgemäßes Konjugat gebunden ist. Erfindungsgemäße Konjugate können an beliebige Zielzellen ex vivo bzw. in vitro angekoppelt werden, sofern die Zielzellen den Bindungspartner der ZK des jeweiligen erfindungsgemäßen Konjugats aufweisen. Die Bindung an die Zielzelle über die ZK kann bspw. spezifisch (s.o.) oder durch die zuvor offenbarten (Aryl)boronsäuren oder (Aryl)boronsäureester an die Zelloberflächen (enzymatisch oder über vicinale Diole) erfolgen.

Für den Fall, daß ein erfindungsgemäßes Addukt als Tumorvakzine verwendet werden soll, sollte die Zielzelle vorzugsweise eine ein Tumor-Antigen präsentierende Zelle sein. Hierbei kann es sich um Zellen, insbesondere humane Zellen und vor allem um humane Zellen des Immunsystems, handeln, die mit Hilfe gentechnologischer Verfahren mit DNA-Abschnitten für mindestens ein membranständiges Antigen, vorzugsweise ein Tumorantigen, transfiziert worden sind. Typischerweise werden die transfizierten Tumor-Antigene für den jeweils zu therapierenden Tumor charakteristisch sein. Bevorzugt ist die Transfektion einer Zelle, die Zielzelle in einem erfindungsgemäßen Addukt sein soll, mit zwei oder drei verschiedenen Antigen, vorzugsweise Tumor-Antigenen. Die für die Transfektion verwendeten, vorzugsweise humanen Zellen, werden ganz besonders bevorzugt dem zu vakzinierenden Patienten entnommen worden sein ("autologe" Zellen), um etwaige Abstoßungsreaktionen des Patienten nach der Vakzinierung auszuschließen.

Weiterhin kann in einer anderen bevorzugten Ausführungsform der vorliegenden Erfindung die Zielzelle in einem erfindungsgemäßen Addukt eine Tumorzelle des zu vakzinierenden Patienten sein, die ohne oder nach zuvor beschriebener Transfektion Zielzelle in einem erfindungsgemäßen Addukt sein kann.

In einer weiteren bevorzugten Ausführungsform wird in einem erfindungsgemäßen Addukt nicht nur mindestens ein erfindungsgemäßes Konjugat einer Klasse an die Zielzelle gebunden sein, sondern es liegen an dieselbe Zielzelle gekoppelt, auch Konjugate mindestens einer anderen Konjugat-Klasse (also Konjugate anderer Struktur) vor. Dies bedeutet, daß in einem erfindungsgemäßen Addukt eine Zielzelle unterschiedliche erfindungsgemäße Konjugate tragen kann. Insbesondere wird es sich dabei um solche Kombinationen von unterschiedlichen Konjugaten (mit jeweils unterschiedlichen Immunmodulatoren) handeln, die die physiologische Immunantwort, d.h. insbesondere die Aktivierung von Effektorzellen, durch ihren biologischen Synergismus verstärken. So kann bspw. ein erfindungsgemäßes Addukt einerseits erfindungsgemäße Konjugate mit einem Cytokin als Immunmodulator, andererseits aber auch Konjugate mit einem co-stimulierenden Immunmodulator auf der Zielzelle aufweisen. Auch Kombinationen von Konjugaten mit verschiedenen Cytokinen, bspw. IFN-γ und IL-2, auf einer Zielzelle zu einem erfindungsgemäßen Addukt sind möglich.

Ein weiterer Erfindungsgegenstand stellt Zusammensetzungen dar, die mindestens ein erfindungsgemäßes Addukt enthalten. Zusammensetzungen der vorgenannten Art können auf der Basis physiologischer Kochsalz-Lösungen beruhen und ggf. auch weitere Komponenten, wie z.B. Adjuvantien, enthalten.

Insgesamt werden im Rahmen der vorliegenden Erfindung daher auch Verfahren, insbesondere Vakzinierungsverfahren, zur Behandlung von Tumoren, insbesondere auch soliden Tumoren, offenbart. Hierbei wird eine Zielzelle, bspw. eine zuvor dem jeweiligen Tumorpatienten entnommene Tumorzelle, die membranständig für den Patiententumor charakteristische Tumor-Antigene aufweist, mit Konjugaten mindestens einer erfindungsgemäßen Konjugat-Klasse zu einem erfindungsgemäßen Zielzellen-Konjugat-Addukt kombiniert. Eine Tumorvakzine, enthaltend dieses Addukt bspw. in Form einer Suspension und ggf. weitere Komponenten, wie z.B. Adjuvantien, wird dann in einem weiteren Verfahrensschritt dem Patienten verabreicht, bspw. auf oralem, typischerweise jedoch auf parenteralem Weg, insbesondere durch subkutane, intravenöse oder intramuskuläre Injektion. Die Verabreichung des Addukts als Tumorvakzine kann ein oder mehrmals erfolgen, bevorzugt mehrfach jeweils in einem Abstand von 7 bis 14 Tagen. In vivo erfolgt dann nach der Injektion über die pH-labile Sollbruchstelle an der Amidbindung (Bindung eines Amins an die heterobifunktionale Brückenverbindung) eine kontrollierte Freisetzung des Immunmodulators aus der Tumorvakzine, insbesondere. unter sauren Bedingungen im extrazellulären Raum. Die immunmodulatorische Wirkung des Immunmodulators entfaltet sich also erst durch Freisetzung unter diesen Bedingungen, insbesondere also im Tumorgewebe, wo diese Bedingungen typischerweise zu finden sind.

Bevorzugt sind die Vakzinierungsverfahren dann, wenn erfindungsgemäße Addukte, die entweder ohne weitere Komponenten oder als Bestandteil einer Zusammensetzung mit anderen Komponenten als Tumorvakzine eingesetzt werden, vor der Applikation bestrahlt werden, um die Lebensfähigkeit der im Addukt auftretenden Zielzelle, insbesondere einer Tumorzelle, zu beeinträchtigen. Insoweit sind erfindungsgemäße Addukte bzw. Zusammensetzungen, enthaltend derartige Addukte, bspw. zum Einsatz als Tumorvakzine, dann ganz besonders bevorzugt, wenn sie zuvor bestrahlt worden sind. Typischerweise erfolgt die Bestrahlung in Abhängigkeit von der jeweiligen Tumorentität mit 1000 bis 15000 rad.

Ganz besonders bevorzugt sind insbesondere für Vakzinierungsverfahren bzw. für die Verwendung der Addukte zur Tumorvakzinierung bzw. für die Verwendung derartiger Addukte zur Herstellung einer Tumorvakzine solche Konjugate im Addukt, die eine unspezifische ZK, insbesondere eine (Aryl)boronsäure oder einen (Aryl)boronsäureester aufweisen. Mit Hilfe der unspezifischen Kopplungsagenzien kann ein erfindungsgemäßes Konjugat an verschiedene Zielzellen, bspw. an Zielzellen von verschiedenen Patienten, angekoppelt werden, ohne - wie im Fall der spezifischen Kopplung über Rezeptor-Liganden-Interaktion - den Nachteil der wiederholten Bereitstellung von jeweils geeigneten Konjugaten mit einer entsprechenden ZK für die jeweils unterschiedlichen membranständigen Bindungspartner auf verschiedenen (Patienten)zielzellen in Kauf nehmen zu müssen. Auf diese Weise können daher unterschiedliche Zielzellen mit demselben Konjugat markiert werden. Im Ergebnis können also mit Hilfe dieser identischen erfindungsgemäßen Konjugate und ihrer Fähigkeit zur unspezifischen Bindung jeweils (in bezug auf die Zielzellen) unterschiedliche, zur Vakzinierung geeignete erfindungsgemäße Addukte zur Verfügung gestellt werden.

Die vorgenannten erfindungsgemäßen Verfahren bzw. Verwendungen von erfindungsgemäßen Addukten oder Zusammensetzungen, enthaltend ein erfindungsgemäßes Addukt, zur Herstellung einer Tumorvakzine zur Behandlung von Tumorerkrankungen sind insbesondere geeignet zur Behandlung von Leukämie-Erkrankungen, vor allem der akuten lymphoblastischen Leukämie und der myeloischen Leukämie bei Kindern, aber auch zur Behandlung der akuten Leukämie des Erwachsenen, der Non-Hodgkin-Lymphome und dem Plasmozyton sowie der Hodgkin-Lymphome. Aber sie erweisen sich auch als zur Behandlung von Hauttumoren, insbesondere dem Melanom und dem Plattenepithelzellkarzinom, sowie zur Behandlung von soliden Tumoren der Lunge, des Darms, insbesondere des Dickdarmkarzinoms, des Magens, der Niere und der Bauchspeicheldrüse, einsetzbar.

Als erfindungsgemäße Konjugate kommen jedoch auch solche substituierten heterofunktionalen substituierten Brückenverbindungen ("cross-linker") in Betracht, an die eine spezifische oder unspezifische Zellerkennungseinheit, insbesondere eine unspezifische ZK, vor allem eine (Arylboron)säure oder ein (Arylboron)säureester, wobei alle bereits zuvor offenbarten Varianten denkbar sind, sowie eine bioaktive Substanz, die keine immunmodulatorische Wirkung besitzt, angekoppelt ist. Ganz besonders bevorzugt sind bioaktive Substanzen, die sich zur Behandlung von Tumoren eignen. Einige der möglichen bioaktiven Substanzen sind nachfolgend beispielhaft aufgeführt. Diese Liste ist aber keineswegs erschöpfend und soll die Erfindung nicht beschränken.

Zu den bioaktiven Substanzen zählen daher insbesondere Wachstumsfaktoren, wie EGF, GCSF, GGF, GMF, GMA, GMCF, IGF, NGF, PDGF, PD-ECGF, TGF oder VDGF, Wachstumsfaktoren des hämatopoetischen Systems, wie z.B. MDGF, SCF, FLT-3L, insbesondere auch weitere Angiogenesefaktoren oder auch Anti-Angiogenesefaktoren, wie z.B. Endostatin oder Angiostatin; Zelldifferenzierungsfaktoren, wie z.B. BMP, TGF, VDGF; auch in der Kontrollkaskade übergeordnete Hormone, z.B. die vom Hypothalamus oder der Hypophyse sekretierten Hormone, wie z.B. Releasing-Hormone, insbesondere GHRH oder TRH, oder ACTH, Somatotropin oder LH. Weiterhin sind als bioaktive Substanzen Faktoren zu nennen, die an der Apoptose beteiligt sind, bspw. FasL, ggf. auch NFκB. Insbesondere sind auch Mimetika der vorgenannten Hormone als bioaktive Substanzen bevorzugt, vor allem dann, wenn sie so modifiziert sind (z.B. synthetisch, oder im Falle von Peptid- oder Proteinhormonen, durch Sequenzveränderungen (Substitutionen, Insertionen und/oder Deletionen), daß sie zwar an ihre physiologischen Rezeptoren binden können, dann aber das physiologische Signal nicht auslösen können. In diesem Fall blockieren die Mimetika der bioaktive Substanzen im erfindungsgemäßen Konjugat die entsprechenden Rezeptoren und sind in vivo damit vorzugsweise kompetitive Inhibitoren der genuinen Liganden.

Bei den bioaktiven Substanzen kann es sich jedoch auch um die Rezeptoren, insbesondere die löslichen Formen der membranständigen Rezeptoren der vorgenannten Liganden handeln. Werden diese Rezeptoren oder Rezeptorfragmente (insbesondere die extrazelluläre(n) Domäne(n) der Rezeptoren) als bioaktive Substanzen in einem erfindungsgemäßen Konjugat bereitgestellt, so können sie in einem geeigneten physiologischen Milieu freigesetzt werden und dort die physiologisch auftretenden Liganden in einer kompetitiven Bindungsreaktion wegfangen. Damit wird die physiologische Signaltransduktion an der Zellmembran attenuiert oder idealerweise inhibiert.

Weiterhin sind unter bioaktiven Substanzen, die in den Konjugaten auftreten können, auch Chemotherapeutika, wie Zytostatika, beispielsweise cis-Platin, Carboplatin, Procarbazin, Mitoxanthron, Doxorubicin, Zorubicin, Epirubicin, Melphalan, Nor-N-lost, Mitomycin C oder Bleomycin, Radiochemotherapeutika oder Enzyminhibitoren zu verstehen; schließlich auch Antibiotika, wie beispielweise die Penicilline, Cephalosporine, Streptomycine und viele andere. Ganz allgemein kommen auch native oder synthetische Peptide mit Inhibitor- oder Aktivatorfunktion für Rezeptoren oder Liganden, Enzyme oder Proteine des Signaltransduktionswegs in Betracht.

Schließlich kommen als bioaktive Substanzen in derartigen Konjugaten auch Faktoren, die im weitesten Sinne die Signaltransduktion oder auch die Zelldifferenzierung beeinflussen, in Frage. Daneben können bioaktive Substanzen auch solche Substanzen sein, die auf die DNA oder RNA von Zellen, insbesondere Tumorzellen, einwirken. So etwa können an erfindungsgemäße Konjugate anti-sense DNA oder RNA oder Ribozyme gekoppelt sein, die für die jeweiligen Tumore spezifische, typischerweise die Hyperproliferation der Zellen steuernde DNA- oder RNA-Sequenzen erkennen, binden und/oder schneiden können. Da derartige Ribozyme oder anti-sense DNA- bzw. RNA vorteilhafterweise intrazellulär wirken, sollte das Konjugat so ausgestaltet sein, daß das anti-sense-Molekül oder das Ribozym in die Tumorzellen eindringen kann.

Typischerweise gibt es zwei Möglichkeiten, die Aufnahme einer bioaktiven Substanz in den intrazellulären Raum sicherzustellen. Wird ein erfindungsgemäßes Konjugat mit einer bioaktiven Substanz oder eine Zusammensetzung, enthaltend ein derartiges Konjugat, systemisch verabreicht, so wird, wie oben beschrieben, die Inkorporation durch eine geeignete Wahl einer ZK sichergestellt. Hierbei bindet die ZK an einen membranständigen Bindungspartner auf der Oberfläche der Zielzelle, wobei der Bindungspartner auf der Zelloberfläche nach Bindung des Konjugats die physiologische Eigenschaft aufweist, internalisiert zu werden.

Wird dagegen ein Addukt aus Zielzelle und Konjugat ex vivo hergestellt und dann dem Patienten verabreicht, so wird die Inkorporation der bioaktiven Substanz im Addukt erfindungsgemäß dadurch gelöst, daß die bioaktive Substanz zwei kovalente Bindungen eingeht: einerseits eine erfindungsgemäß säurelabile Bindung an einen heterobifunktionalen "cross-linker", andererseits eine weitere Bindung unmittelbar an eine weitere ZK. Diese weitere ZK ist so gewählt, daß sie als Bindungspartner ein membranständiges Oberflächenmolekül von bspw. Tumorzellen aufweist, wobei das Oberflächenmolekül nach Bindung seines Bindungspartners die Fähigkeit zur Internalisierung besitzt. Bspw. wird also ein derart konstruiertes Addukt unter entsprechenden sauren Bedingungen, bspw. im Tumorgewebe, nach Spaltung der säurelabilen Bindung zwischen "cross-linker" und bioaktiver Substanz die bioaktive Substanz im extrazellulären Raum freisetzen. Diese im extrazellulären Raum lokalisierte, bioaktive Substanz ist allerdings kovalent mit einer weiteren ZK verbunden. Über diese ZK kann die bioaktive Substanz an den entsprechenden Bindungspartner auf der Zielzelloberfläche, typischerweise eine Tumorzelle, binden und wird auf Grund dann auf Grund der Eigenschaften des Bindungspartners in den intrazellulären Raum aufgenommen. Auf diese Weise. können intrazellulär wirkende bioaktive Substanzen, bspw. auch Ribozyme oder anti-sense-Moleküle, auch nach Verabreichung von erfindungsgemäßen Addukten in Zellen eingeschleust werden.

Daher ist es möglich, Konjugate mit einer bioaktiven Komponente im obigen Sinne, als Arzneimittel, zur Verwendung als Arzneimittel bzw. zur Verwendung zur Herstellung von Arzneimitteln zur Behandlung von Tumorerkrankungen (insbesondere anti-sense-Moleküle oder Ribozyme, die vorgenannten Chemotherapeutika oder die vorgenannten kompetitiven Mimetika von Wachstumsfaktoren), bakteriellen Infektionen (vorgenannte Antibiotika), zur Beschleunigung der Wund- und Frakturheilung (vorgenannte Wachstumsfaktoren), zur Gefäßbildung (Angiogenese-Faktoren), zur Genese von Geweben, bspw. für den Organersatz, oder zur Verhinderung der Gefäßbildung (vorgenannte Anti-Angiogenesefaktoren, die auch zur Tumorbekämpfung geeignet sind, einzusetzen.

Für Konjugate, die eine bioaktive Substanz aufweisen, wobei die bioaktive Substanz nicht immunmodulatorisch wirkt, gilt die gesamte vorhergehende Offenbarung zu immunmodulatorisch wirkenden, erfindungsgemäßen Konjugaten und deren bevorzugten Ausführungsformen entsprechend. Auch auf die Offenbarung zu Zusammensetzungen, die diese Konjugate aufweisen, oder auch zu Addukten aus Konjugaten und Zielzellen wird entsprechender Bezug genommen, soweit die Konjugate bioaktive Substanzen im obigen Sinne enthalten.

Die vorliegende Erfindung wird durch die nachfolgenden Ausführungsbeispiele näher erläutert:

### Beispiel 1 : Synthese eines erfindungsgemäßen Konjugats mit einer heterobifunktionalen Brückenverbindung (cross-linker), einer zellerkennenden Einheit (ZK) und einem Immunmodulator der allgemeinen Formel (II):

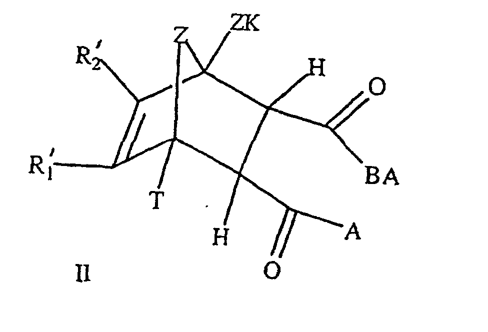

in der: R'₁ = R'₂ = T = H; Z = O; ZK = B(OH)₂; A = OH; BA = IL-2
**a)** Synthese des Maleinsäureanhydrid-Addukts von 2-Furylboronsäure
   10,000 g (0,084 Mol) 2-Furylboronsäure (Aldrich) werden in 100 ml Diethylether/THF (1:1) gelöst. Danach werden 8,064 g (0,084 Mol) Maleinsäureanhydrid dazugegeben. Nach 20 Stunden wird die Reaktionslösung unter vermindertem Druck vorsichtig eingedampft. Man erhält so 15 g (83,3%) des Diels-Alder-Adduktes.
   IR (KBr) = 1895 cm⁻¹.
**b)** Synthese des Interleukin-Konjugats
   100 µg Interleukin-2 (IL-2, Calbiochem) werden in 20 µl 10 mM Phosphat, pH 7,5, mit 10 µg des in a) hergestellten Diels-Alder-Adduktes 30 Minuten bei Raumtemperatur inkubiert. Anschließend wird die Reaktionslösung über einen Amino-dotierten Mikrocellulosefilter in eine 1%-ige Human-Serumalbumin-(HSA) -Lösung filtriert, um überschüssiges reaktives Anhydridmaterial zu entfernen, und dann portioniert tiefgefroren.

### Beispiel 2: Synthese eines erfindungsgemäßen Konjugats mit einer heterobifunktionalen Brückenverbindung, zwei zellerkennenden Einheiten und einem Immunmodulator der allgemeinen Formel (II):

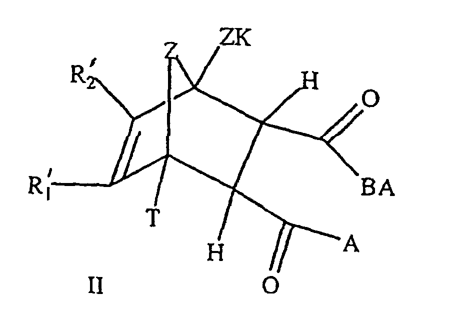

in der: R'₁ = R'₂ = H; Z = O; ZK = B(OH)₂; T = CH₂-N(C(O)CH₃)(C₆H₄)-meta-B(OH)₂; A = OH; BA = IL-2
**a)** Synthese der Ausgangsverbindung 5-[N-(3-Dihydroxyborylphenyl)-N-acetylaminomethyl]-2-furylboronsäure
   5 g 2-Furyl-5-formylboronsäure (0,035 Mol) (Aldrich) werden mit 6,22 g (0,035 Mol) 3-Aminophenylboronsäure (Aldrich) und einem Äquivalent Triethylamin versetzt und in der Schmelze kondensiert. Danach wird das Rohprodukt in 10 ml Wasser und 10 ml Methanol suspendiert und mit einem Überschuß Natriumcyanborhydrid versetzt und 2 Tage bei Raumtemperatur gerührt. Danach wird der pH-Wert auf pH 8 eingestellt und das Rohprodukt mit Dichlormethan extrahiert. Die Lösungsmittel werden eingedampft, und der Rückstand wird über Nacht mit Pyridin/Acetanhydrid behandelt. Anschließend wird das Lösungsmittel eingedampft, der Rückstand mit Wasser aufgenommen und mit Ethylacetat extrahiert. Man erhält nach Umkristallisieren 1,8 g (17%) der gewünschten Verbindung.
**b)** Synthese des Maleinsäureanhydrid-Adduktes von 5-[N-(3-Dihydroxyborylphenyl) -N-acetylamino] -2-furylboronsäure
   1,000 g (0,003 Mol) des in a) hergestellten Bisboronsäure-Derivats werden mit 10 ml Diethylether/THF (1:1) gelöst. Danach werden 0,324 g (0,003 Mol) Maleinsäureanhydrid dazugegeben. Man rührt 20 Stunden, dann wird die Reaktionslösung vorsichtig unter vermindertem Druck eingedampft. Man erhält so 1,3 g (95%) des Diels-Alder-Adduktes mit zwei zellerkennenden Systemen ZK.
   IR (KBr) = 1898 cm⁻¹.
**c)** Synthese des Interleukin-2-Konjugats
   100 µg Interleukin-2 (Calbiochem) werden in 20 µl 10 mM Phosphat, pH 7,5, mit 10 µg des in b) hergestellten Diels-Alder-Adduktes 30 Minuten bei Raumtemperatur inkubiert. Anschließend wird die Reaktionslösung über einen Amino-dotierten Mikrocellulosefilter in eine 1%-ige HFA-Lösung filtriert (um überschüssiges Anhydridreagenz zu entfernen) und dann portioniert tiefgefroren.

### Beispiel 3: Herstellung eines erfindungsgemäßen Addukts aus einem erfindungsgemäßen Konjugat nach Beispiel 1 oder 2 und einer Zielzelle

Erfindungsgemäße Konjugate nach den Ausführungsbeispielen 1 oder 2 wurden an (i) murine oder an (ii) humane Tumorzellen neuronalen oder hämatopoetischen Ursprungs gebunden. Hierbei wurde zunächst ein Anhydrid mit einer Arylboronsäure als ZK und einem Zellmarker (Fluorescein) oder - in einem parallelen Ansatz - einem Immunmodulator (IL-2) zu heterobifunktionalen Konjugaten umgesetzt.
Zum Nachweis der Zellbindung wurden 100 µM des Fluorescein-markierten Konjugats mit 2x10⁵ Zielzellen in einem Volumen von 1 ml in einem 15ml-Röhrchen inkubiert. Die Zellbindung wurde zu unterschiedlichen Zeitpunkten nach Beginn der Inkubation in einem Durchflußzytometer anhand der Intensität der gemessenen Fluoreszenz bestimmt.

Die Bindung des Moleküls an die Zielzellen erfolgte innnerhalb weniger Minuten, wobei durch Fluoreszenzmarkierung der Arylboronsäure als ZK festgestellt werden konnte, daß nach 5 min bereits alle Zellen (100 %) markiert waren. Diese Bindung der erfindungsgemäßen Konjugate an die Zielzellen in allen vier Ansätzen (murine Tumorzellen neuronalen Ursprungs, murine Tumorzellen hämatopoetischen Ursprungs, human Tumorzellen neuronalen Ursprungs und humane Tumorzellen hämatopoetischen Ursprungs) blieb bei Temperaturen von 4°C und bei physiologischen Temperaturen von 37°C stabil.

In den vier vorgenannten Zellkulturen der erfindungsgemäßen Addukte konnte keine gegenüber Vergleichszellkulturen (die vorgenannten Tumorzellen ohne Ankopplung eines erfindungsgemäßen Konjugats) veränderte Zellproliferation beobachtet werden. Auch wurden bei den erfindungsgemäßen Addukten keine apoptotischen Prozesse, keine veränderte Zelladhäsion und auch keine veränderte Antigen-Präsentation festgestellt. Die Ergebnisse wurden durch entsprechende dem Fachmann geläufige Standardtest erhalten, bspw. die Untersuchungen zur Apoptose mit Hilfe des Propidiumiodid-Nachweises mit nachfolgender Durchflußzytometrie bzw. mit Antikörper-Färbung, wobei die Antikörper T-Zellen erkennen, und nachfolgender Detektion mittels Durchflußzytometrie.

### Beispiel 4: Freisetzung des Immunmodulators in vitro

Die nach Beispiel 3 erhaltenen erfindungsgemäßen Addukte aus Konjugat und Tumorzielzelle wurden in Kultur genommen. Im Verlauf der ersten 12 Stunden der Kultivierung wurden jeweils im Abstand von zwei Stunden Überstände aus der Zellkultur entnommen, danach im Abstand von 12 Stunden. Die in diesen Überständen enthaltene Konzentration an freigesetztem Immunmodulator, im vorliegenden Fall also IL-2, wurde mittels der ELISA (enzyme linked immuno assay) ermittelt.

Hierbei wurde festgestellt, daß von 106 Zellen in einem Zeitraum von 24 h ≥ 500 pg IL-2 freigesetzt wurde, wobei eine Halbwertszeit des Konjugats von 100 h eingestellt wurde.

### Beispiel 5: Tumorvakzinierung mit einem erfindungsgemäßen Addukt nach Beispiel 3

Die gemäß Beispiel 3 erhaltenen Addukte mit murinen Tumorzellen hämatopoetischen Ursprungs (Leukämiezellen) wurden jeweils als Tumorvakzine im Mausmodell getestet. Das Tumormodellsystem wurde dadurch etabliert, daß Mäusen subkutan Leukämiezellen injiziert wurden. Das Anwachsen des Tumors im subkutanen Gewebe vor und nach Vakzination wurde durch Messungen im Abstand von drei Tagen verfolgt.

Drei bzw. 10 Tage nach der subkutanen Injektion der Leukämiezellen wurde die derart vorbehandelten Mäuse mit der Tumorvakzine (s. Bsp. 3) behandelt. Hierzu wurde die Tumorvakzine subkutan injiziert, wobei die in der Tumorvakzine enthaltenen Leukämiezellen zuvor bestrahlt wurden. Als Kontrollversuch wurden nicht an ein erfindungsgemäßes Konjugat gekoppelte Leukämiezellen, ebenfalls bestrahlt, in der gleichen Weise wie die Tumorvakzine der Maus appliziert.

Hierbei ergab sich, daß bei dem weit überwiegenden Teil der vakzinierten Mäuse eine Unterdrückung des Tumorwachstums feststellbar war, was sich in einem deutlich erhöhten Langzeitüberleben äußerte. Die durch die Vakzination erhaltene Immunabwehr wird durch T-Zellen vermittelt, wie sich anhand von Depletionen immunologischer Effektozellpopulationen in vivo nachweisen ließ.

## Patentansprüche

1. Verbindungen der allgemeinen Formel (I) und (II) 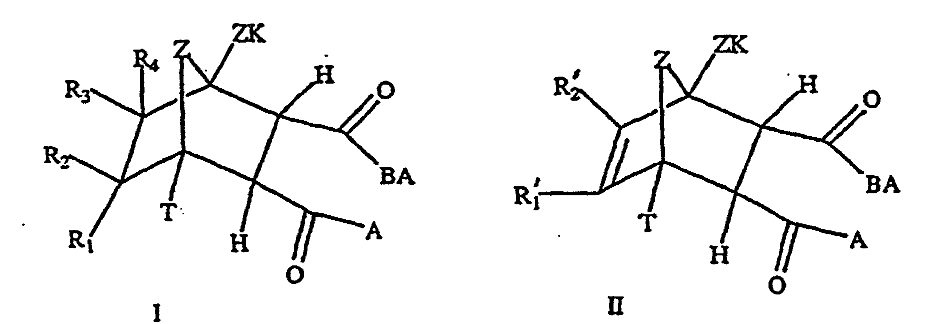 worin
R₂ und R₄, für Wasserstoff oder gemeinsam für -O- oder -NR₁₀ stehen, worin R₁₀ geradkettiges oder verzweigtes Alkyl mit bis zu 6 Kohlenstoffatomen ist,
R₁, R₃, R'₁ und R'₂ unabhängig voneinander für Wasserstoff oder eine lineare, verzweigte oder cyclische, gegebenenfalls substituierte Alkylgruppe mit 3 bis 8 Kohlenstoffatomen stehen, wobei die Substituenten vorzugsweise aus Halogen, Amino, Cyano, Carboxy, geradkettigem oder verzweigtem Alkoxy mit bis zu 6 Kohlenstoffatomen oder Hydroxy ausgewählt sind;
oder R₁ und R₃ bzw. R'₁ und R'₂ gemeinsam entweder einen 5- bis 7-gliedrigen ungesättigten Heterocyclus mit bis zu 3 Heteroatomen aus der Reihe N, S oder O oder einen 4- bis 6-gliedrigen Carbocyclus bilden;
Z für Sauerstoff, Schwefel, -NR₅-, -CR₆R₇- oder -CR₆R₇-CR₈R₉- steht, worin R₅ für H oder C₁₋₈-Alkyl steht und R₆, R₇, R₈ und R₉ unabhängig für H oder C₁₋₆-Alkyl stehen;
A für Hydroxy steht,
BA (Komponente 2) für einen Immunmodulator steht, der eine primäre oder sekundäre Aminfunktion aufweist, und der ein Cytokin, ein Chemokin, ein Monokin, ein Lymphokin, ein Interferon, ein an der Immunregulation beteiligtes Zelloberflächenprotein und/oder ein an der Aktivierung der vorgenannten Immunmodulatoren beteiligter Transkriptionsfaktor bzw. ein Derivat oder Fragment derselben ist,
ZK (Komponente 3) für eine mit dem Rest der allgemeinen Formeln I oder II kovalent verknüpfte zellerkennende Einheit mit spezifischer oder unspezifischer Zellerkennung steht,
T unabhängig die Bedeutung von ZK aufweist oder für H, eine lineare, verzweigte oder cyclische, gegebenenfalls substituierte Alkylgruppe mit 3 bis 8 Kohlenstoffatomen steht, wobei die Substituenten vorzugsweise aus einem Halogen, Amino-, Cyano-, Carboxy- oder Zuckerrest ausgewählt sind.

2. Verbindung nach Anspruch 1 der Formel (II), wobei R₁, R₂ und T für H stehen, Z für O steht, A für OH steht, BA für einen Immunmodulator steht und ZK für -B(OH)₂ steht.

3. Verbindung nach Anspruch 1 der Formel (II), wobei R₁ und R₂ für H stehen, T für -CH₂-N(C(O)-meta-C₆H₄-B(OH)₂ steht, Z für O steht, ZK für B(OH)₂ steht, A für OH steht und BA für einen Immunmodulator steht.

4. Verbindung nach einem der Ansprüche 1 bis 3, **dadurch gekennzeichnet, daß** der Immunmodulator ein Interleukin, insbesondere IL-2, ist.

5. Verbindung nach einem der vorgenannten Ansprüche 1 bis 4, wobei ZK ein Antikörper, ein Lektin oder eine Kohlenhydratverbindung, ein Bindungspartner eines membranständigen Rezeptors oder Liganden bzw. ein Fragment oder Derivat einer der vorgenannten Substanzen, ein Liposom oder eine zellerkennende (Aryl)boronsäure ist.

6. Verbindung nach einem der vorgenannten Ansprüche 1 bis 5, wobei an die Verbindung, insbesondere an die Komponente (2) (den Immunmodulator), mindestens eine weitere Substanz kovalent angekoppelt ist.

7. Verbindung nach Anspruch 6, wobei die mindestens eine weitere Substanz eine bioaktive Verbindung ist, bspw. ein Wachstumsfaktor, identischer oder divergenter Immunmodulator, ein Angiogenese- oder Anti-Angiogenese-Faktor, Signaltransduktions-Inhibitor, Enzyminhibitor, eine Apoptose-induzierende Verbindung, Antibiotikum, Chemotherapeutikum, Radiochemotherapeutikum und/oder ein Markermolekül, bspw. ein Fluoreszenzmarker.

8. Verbindung nach einem der Ansprüche 1 bis 7, wobei die Verbindung multimerisiert, insbesondere di-, tri-, tetra- oder pentamerisiert, ist.

9. Zusammensetzung, enthaltend eine Verbindung nach einem der Ansprüche 1 bis 8.

10. Arzneimittel, enthaltend eine Verbindung nach einem der Ansprüche 1 bis 8.

11. Verwendung einer Verbindung nach einem der Ansprüche 1 bis 8 oder eines Arzneimittels nach Anspruch 10 zur Herstellung eines Arzneimittels zur Behandlung von Tumorerkrankungen, insbesondere Leukämien, Autoimmunerkrankungen oder Infektionserkrankungen bzw. zur Herstellung eines Arzneimittels zur Behandlung der vorgenannten Erkrankungen.

12. Verfahren zur Herstellung einer Verbindung nach einem der Ansprüche 1 bis 8 der allgemeinen Formel (I) oder (II) 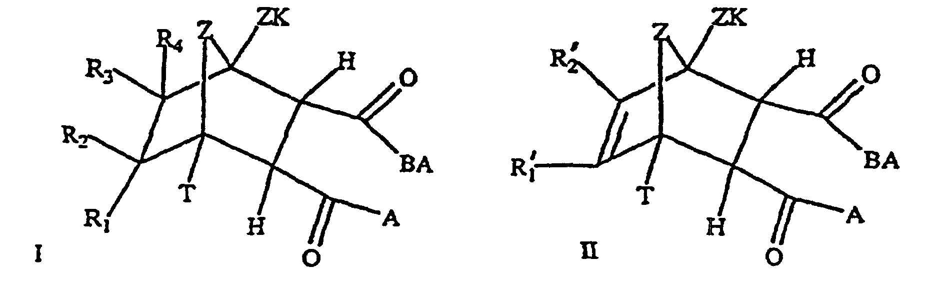 worin
R₁, R₃, R'₁, R'₂ A, T, Z, ZK und BA die in Anspruch 1 aufgeführten Bedeutungen aufweisen und R₂ und R₄ für H stehen,
welches umfaßt:
a) Umsetzten eines Diens der Formel (III) 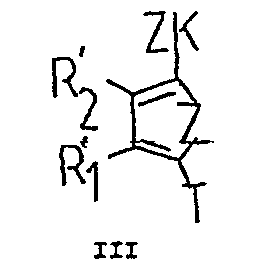 in der R'₁, R'₂, T, Z und ZK die in Anspruch 1 äufgeführten Bedeutungen aufweisen, mit Maleinsäureanhydrid, um ein Produkt der Formel (IV) zu erhalten: 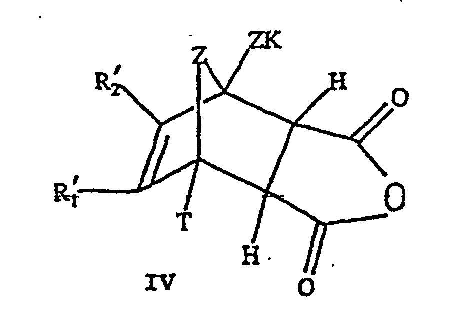 in der R'₁, R'₂, T, Z und ZK die in Anspruch 1 aufgeführten Bedeutungen aufweisen,
b) gegebenenfalls Hydrieren des Produktes der Formel (IV) mit einem Hydrierkatalysator, um ein Produkt der Formel (V) zu erhalten: 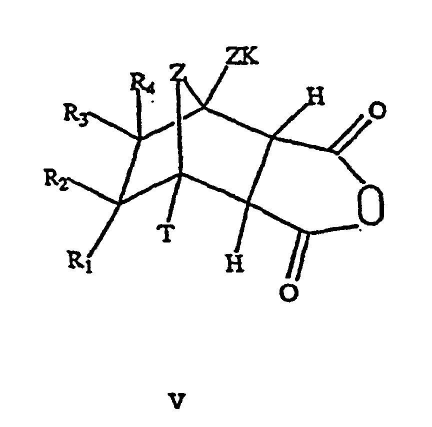 in der R₁, R₃, T, Z und ZK die in Anspruch 1 aufgeführten Bedeutungen aufweisen und R₂ und R₄ für H stehen, und
c) Inkubieren des Produktes der Formel (IV) oder (V) mit einer biospezifischen Verbindung, die eine primäre oder sekundäre Aminfunktion enthält, um ein Produkt der Formel I oder II zu erhalten.

13. Addukt aus Verbindungen nach einem der Ansprüche 1 bis 8 und einer Zelle, wobei die Verbindung(en) über ihre ZK spezifisch oder unspezifisch an die Zelle gekoppelt sind/ist.

14. Addukt nach Auspruch 13, wobei die Zelle eine Tumorzelle, eine humane Immunzelle, insbesondere eine dendritische Zelle bzw. ein B- oder ein T-Lymphozyt, ist.

15. Addukt nach Anspruch 13 oder 14, wobei an die Zelle Verbindungen mindestens zwei verschiedener Klassen, insbesondere mit unterschiedlichen Immunmodulatoren, angekoppelt sind.

16. Zusammensetzung, enthaltend Addukte gleicher oder unterschiedlicher Art nach einem der Ansprüche 13 bis 15 und ggf. weitere Bestandteile.

17. Verwendung eines Addukts nach einem der Ansprüche 13 bis 15 bzw. einer Zusammensetzung nach Anspruch 16 zur Herstellung einer Tumorvakzine.

## Claims

1. Compounds of the general formula (I) and (II) 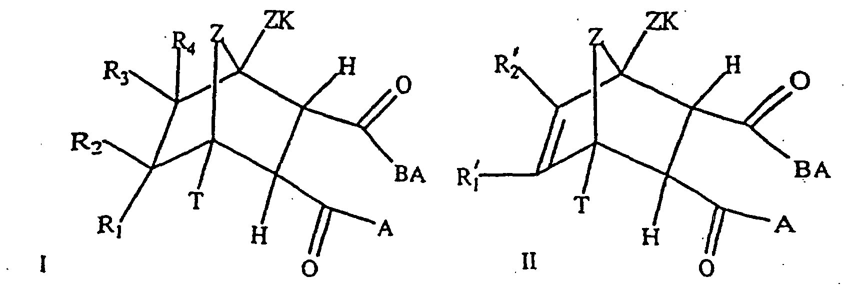 where
R₂ and R₄ are hydrogen, or together form -O- or -NR₁₀-, where R₁₀ is a linear chain or branched alkyl having up to 6 carbon atoms,
where R₁, R₃, R'₁ and R'₂ are independently hydrogen or a linear, branched or cyclic, optionally substituted alkyl group having 3 to 8 carbon atoms, where the substituents are preferably selected from halogen, amino, cyano, carboxy, linear chain or branched alkoxy having up to 6 carbon atoms, or hydroxy;
or where R₁ and R₃, or R'₁ and R'₂ together form either a 5- to 7-segment unsaturated heterocycle having up to 3 heteroatoms selected from N, S, and O, or a4- to 6-segment carbocycle;
where Z is oxygen, sulfur, -NR₅-, -CR₆R₇-, or -CR₆R₇-CR₈R₉-, where R₅ is H or C₁₋₈-alkyl, and R₆, R₇, R₈ and R₉ are independently H or C₁₋₆-alkyl;
where A is hydroxy;
where BA (component 2) is an immunomodulator having a primary or secondary amine group, and being a cytokine, a chemokine, a monokine, a lymphokine, an interferon, a cell surface protein involved in immune regulation, and/or a transcription factor involved in the activation of the above-mentioned immunomodulators, or a derivative or fragment of same,
where ZK (component 3) is a cell recognising unit, covalently linked with the remainder of the general formula I or II, having specific or unspecific cell recognition,
where T, independently, is as defined for ZK, or is H, a linear, branched or cyclic, optionally substituted alkyl group having 3 to 8 carbon atoms, where the substituents are preferably selected from a halogen, amino, cyano, carboxy or sugar residue.

2. The compound according to claim 1 of formula (II), where R₁, R₂ and T is H, where Z is O, where A is OH, where BA is an immunomodulator, and where ZK is -B(OH)₂.

3. The compound according to claim 1 of formula (II), where R₁ and R₂ is H, where T is -CH₂-N(C(O)-meta-C₆H₄-B(OH)₂, where Z is O, where ZK is -B(OH)₂, where A is OH, and where BA is an immunomodulator.

4. The compound according to one of the claims 1 to 3, **characterised in that** the immunomodulator is an interleukin, especially IL-2.

5. The compound according to one of the above-mentioned claims 1 to 4, where ZK is an antibody, a lectin or a carbohydrate compound, a binding partner of a membrane-residing receptor or ligand, or a fragment or derivative of the above-mentioned substances, a liposome or a cell recognising (aryl) boronic acid.

6. The compound according to one of the above-mentioned claims 1 to 5, where at least one other substance is covalently coupled to the compound, especially to component (2) (the immunomodulator).

7. The compound according to claim 6, where the at least one other substance is a bio-active compound, eg. a growth factor, an identical or divergent immunomodulator, an angiogenesis or anti-angiogenesis factor, a signal transinduction inhibitor, an enzyme inhibitor, an apoptosis inducing compound, an antibiotic, a chemotherapeutic, a radiochemotherapeutic and/or a marker molecule, eg. a fluorescence marker.

8. The compound according to one of the claims 1 to 7, where the compound is multimerised, especially di-, tri-, tetra- or pentamerised.

9. A composition containing a compound according to one of the claims 1 to 8.

10. A medicament containing a compound according to one of the claims 1 to 8.

11. Use of a compound according to one of the claims 1 to 8, or of a medicament according to claim 10 for the production of a medicament for the treatment of tumour diseases, especially leukemia diseases, auto immune diseases or infectious diseases, or for production of a medicament for treatment of the above-mentioned diseases.

12. A method for the production of a compound according to one of the claims 1 to 8 of the general formula (I) or (II) 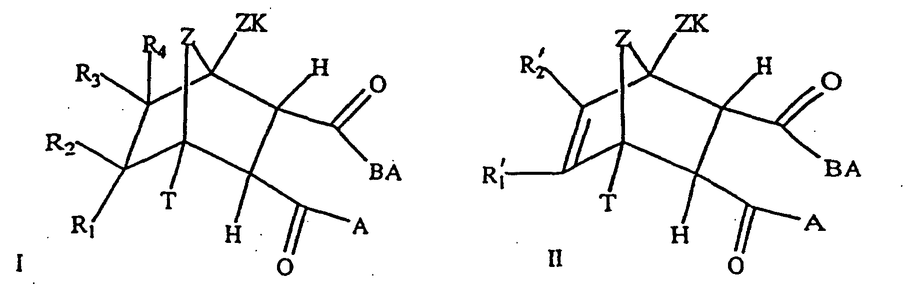 where
R₁, R₃, R'₁, R'₂, A, T, Z, ZK and BA are as defined in claim 1, and where R₂ and R₄ stand for H,
which comprises:
a) Transformation of a diene of the formula (III) 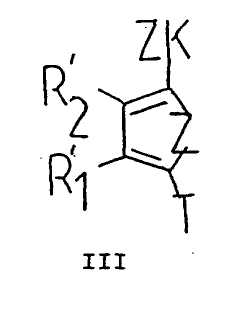 where R'₁, R'₂, T, Z and ZKare as defined in claim 1, with maleic acid anhydride, in order to obtain a product of formula (IV): 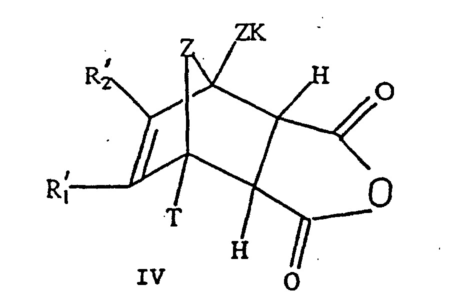 where R'₁, R'₂, T, Z and ZK are as defined in claim 1,
b) Optionally hydrating the product of formula (IV) with a hydration catalyst in order to obtain a product of formula (V): 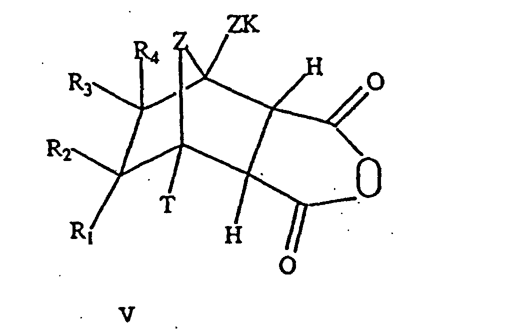 where R₁, R₃, T, Z and ZK are as defined in claim 1, and where R₂ and R₄ stand for H, and
c) Incubating the product of formulas (IV) or (V) with a bio-specific compound containing a primary or secondary amine group in order to obtain a product of formulas I or II.

13. An adduct from compounds according to one of the claims 1 to 8 and a cell, where the compound(s) is/are coupled specifically or unspecifically to the cell via their ZK.

14. The adduct according to claim 13, where the cell is a tumour cell, a human immune cell, especially a dendritic cell or a B- or a T-lymphocyte.

15. The adduct according to claim 13 or 14, where compounds of at least two different classes, especially having different immunomodulators, are coupled to the cell.

16. A composition containing adducts of identical or different type according to one of the claims 13 to 15, and optionally other components.

17. Use of an adduct according to one of the claims 13 to 15, or use of a composition according to claim 16 for the production of a tumour vaccine.

## Revendications

1. Composés de formules générales (I) et (II) 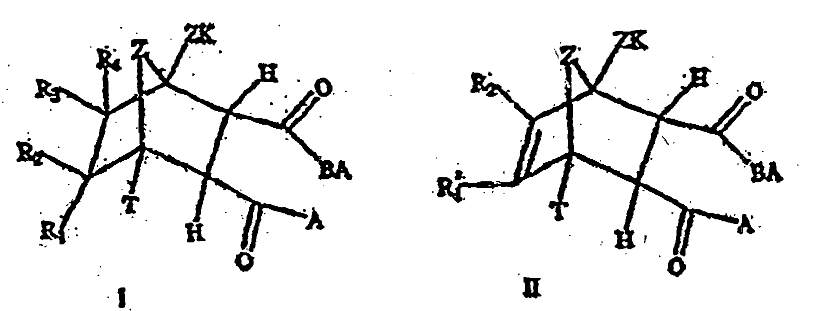 où
R₂ et R₄ représentent l'hydrogène ou ensemble -O- ou -NR₁₀-, où R₁₀ est alkyle linéaire ou ramifié avec jusqu'à 6 atomes de carbone,
R₁, R₃, R'₁ et R'₂ représentent indépendamment les uns des autres l'hydrogène ou un groupe alkyle linéaire, ramifié ou cyclique, éventuellement substitué, avec 3 à 8 atomes de carbone, où les substituants sont choisis de préférence parmi halogène, amino, cyano, carboxyle, alcoxy linéaire ou ramifié avec jusqu'à 6 atomes de carbone ou hydroxyle ;
ou R₁ et R₃ ou R'₁ et R'₂ forment ensemble un hétérocycle insaturé à 5 à 7 chaînons avec jusqu'à 3 hétéroatomes de la série N, S ou O ou un carbocycle à 4 à 6 chaînons ;
Z représente l'oxygène, le soufre, -NR₅-, -CR₆R₇- ou -CR₆R₇-CR₈R₉-, où R₅ représente H ou C₁₋₈-alkyle et R₆, R₇, R₈ et R₉ représentent indépendamment H ou C₁₋₆-alkyle ;
A représente hydroxyle,
BA (composant 2) représente un immunomodulateur qui comporte une fonction amine primaire ou secondaire, et qui est une cytokine, une chimiokine, une monokine, une lymphokine, un interféron, une protéine de la surface cellulaire impliquée dans l'immunorégulation et/ou un facteur de transcription impliqué dans l'activation des immunomodulateurs précités, ou un dérivé ou fragment de ceux-ci,
ZK (composant 3) représente une unité reconnaissant les cellules liée par liaison covalente au groupement de formule générale I ou II avec une reconnaissance des cellules spécifique ou non spécifique,
T indépendamment présente la signification de ZK ou représente H, un groupe alkyle linéaire, ramifié ou cyclique, éventuellement substitué, avec 3 à 8 atomes de carbone, où les substituants sont choisis de préférence parmi un halogène, un groupement amino, cyano, carboxyle ou sucre.

2. Composé selon la revendication 1 de formule (II) où R₁, R₂ et T représentent H, Z représente O, A représente OH, BA représente un immunomodulateur et ZK représente -B(OH)₂.

3. Composé selon la revendication 1 de formule (II) où R₁ et R₂ représentent H, T représente -CH₂-N(C(O)-méta-C₆H₄-B(OH)₂, Z représente O, ZK représente -B(OH)₂, A représente OH et BA représente un immunomodulateur.

4. Composé selon l'une des revendications 1 à 3 **caractérisé en ce que** l'immunomodulateur est une interleukine, en particulier IL-2.

5. Composé selon l'une des revendications 1 à 4 précitées où ZK est un anticorps, une lectine ou un composé glucidique, un partenaire de liaison d'un récepteur membranaire ou d'un ligand ou un fragment ou dérivé de l'une des substances précitées, un liposome ou un acide (aryl)boronique reconnaissant les cellules.

6. Composé selon l'une des revendications 1 à 5 précitées où au moins une autre substance est couplée par liaison covalente au composé, en particulier au composant (2) (l'immunomodulateur).

7. Composé selon la revendication 6 où l'autre ou les autres substances est ou sont un composé bioactif, par exemple un facteur de croissance, un immunomodulateur identique ou divergent, un facteur d'angiogenèse ou anti-angiogenèse, un inhibiteur de transduction de signal, un inhibiteur d'enzyme, un composé induisant l'apoptose, un antibiotique, un agent chimiothérapeutique, un agent radiochimiothérapeutique et/ou une molécule marqueur, par exemple un marqueur de fluorescence.

8. Composé selon l'une des revendications 1 à 7 où le composé est multimérisé, en particulier di-, tri-, tétra- ou pentamérisé.

9. Composition contenant un composé selon l'une des revendications 1 à 8.

10. Médicament contenant un composé selon l'une des revendications 1 à 8.

11. Utilisation d'un composé selon l'une des revendications 1 à 8 ou d'un médicament selon la revendication 10 pour la production d'un médicament pour le traitement des maladies tumorales, en particulier des leucémies, des maladies auto-immunes ou des maladies infectieuses ou pour la production d'un médicament pour le traitement des maladies précitées.

12. Procédé de production d'un composé selon l'une des revendications 1 à 8 de formule générale (I) ou (II) 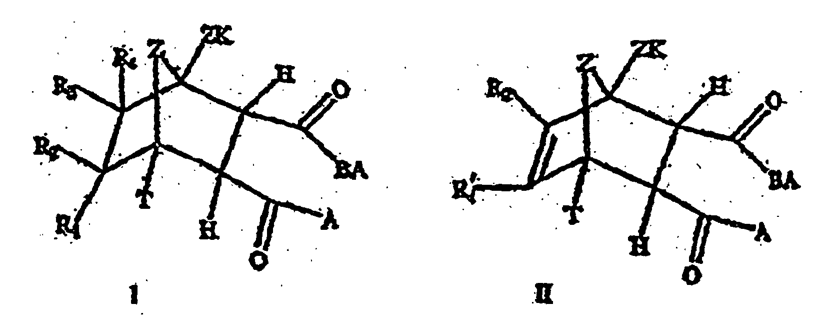 où
R₁, R₃, R'₁, R'₂, A, T, Z, ZK et BA présentent les significations indiquées dans la revendication 1 et R₂ et R₄ représentent H,
qui comprend:
a) la réaction d'un diène de formule (III) 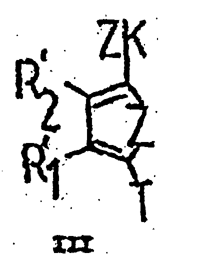 où R'₁, R'₂, T, Z et ZK présentent les significations indiquées dans la revendication 1, avec l'anhydride maléique, pour obtenir un produit de formule (IV): 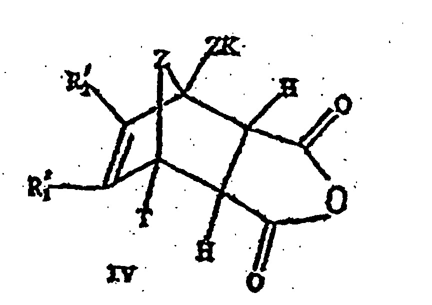 où R'₁, R'₂, T, Z et ZK présentent les significations indiquées dans la revendication 1,
b) éventuellement l'hydrogénation du produit de formule (IV) avec un catalyseur d'hydrogénation, pour obtenir un produit de formule (V) : 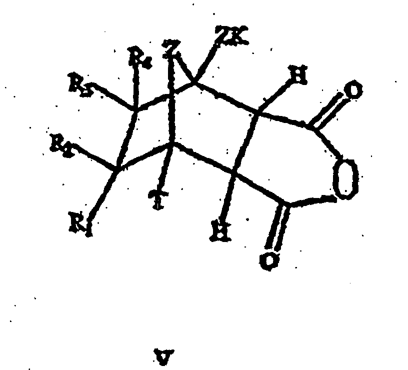 où R₁, R₃, T, Z et ZK présentent les significations indiquées dans la revendication 1 et R₂ et R₄ représentent H, et
c) l'incubation du produit de formule (IV) ou (V) avec un composé biospécifique qui contient une fonction amine primaire ou secondaire, pour obtenir un produit de formule I ou II.

13. Produit d'addition de composés selon l'une des revendications 1 à 8 et d'une cellule, où le ou les composés est/sont couplés spécifiquement ou non spécifiquement à la cellule par le biais de leur ZK.

14. Produit d'addition selon la revendication 13 où la cellule est une cellule tumorale, une cellule immunitaire humaine, en particulier une cellule dendritique ou un lymphocyte B ou T.

15. Produit d'addition selon la revendication 13 ou 14 où des composés d'au moins deux classes différentes, en particulier avec des immunomodulateurs différents, sont couplés à la cellule.

16. Composition contenant des produits d'addition de type identique ou différent selon l'une des revendications 13 à 15 et éventuellement d'autres constituants.

17. Utilisation d'un produit d'addition selon l'une des revendications 13 à 15 ou d'une composition selon la revendication 16 pour la production d'un vaccin anti-tumoral.
